# EUROPEAN PATENT APPLICATION

(11) **EP 3 298 904 A1**
(43) Date of publication of application: **28.03.2018**
(21) Application number: 16796544.1
(22) Date of filing: 18.05.2016
(51) Int. Cl.: A23C 9/127

(54) **METHOD FOR PRODUCING FERMENTED MILK**

(30) Priority: 18.05.2015 JP 2015101071
(71) Applicant: Godo Shusei Co., Ltd., Tokyo 104-0061 (JP)
(72) Inventor: MORIE, Kyoko, Matsudo-shi Chiba 271-0064 (JP); SHINADA, Atsuko, Matsudo-shi Chiba 271-0064 (JP); HORIGUCHI, Hirofumi, Matsudo-shi Chiba 271-0064 (JP); YOSHIKAWA, Jun, Matsudo-shi Chiba 271-0064 (JP)
(74) Representative: Hoffmann Eitle
(86) International application number: PCT/JP2016/064804
(87) International publication number: WO 2016/186151

(57) **Abstract**

[Object] The purpose is to produce fermented milk containing lactic acid bacteria and bifidobacteria while increasing and maintaining the number of live bifidobacteria by a simple method.

[Solving Means] A method for producing fermented milk in which a first step for mixing raw material milk, lactic acid bacteria, and bifidobacteria and a second step for fermenting the raw material milk are carried out in order, wherein a step for adding lactase to the raw material milk (lactase addition step) is carried out before the completion of the second step and the lactase addition step is carried out at one or more time point selected from before the first step, almost simultaneously with the first step, or after the first step.

## Description

### Technical Field

The present invention relates to a method for producing fermented milk using lactic acid bacteria and bifidobacteria.

### Background Art

Lactic acid bacteria or bifidobacteria constituting human intestinal bacterial flora are known to exhibit a favorable activity in the intestine. However, the proliferation rate of lactic acid bacteria or bifidobacteria is slower than that of other microbes constituting intestinal bacterial flora. As such, an invention for selective proliferation of lactic acid bacteria or bifidobacteria in intestine has been suggested (see, Patent Literature 1, for example). Because bifidobacteria have a particularly slow proliferation rate, an invention for specific proliferation of bifidobacteria has been also suggested (see, Patent Literature 2, for example).

As a method for increasing the ratio of lactic acid bacteria or bifidobacteria constituting intestinal bacterial flora, intake of fermented milk which contains lactic acid bacteria and/or bifidobacteria in their living state is also considered. However, from the viewpoint that bifidobacteria are weaker to oxygen and have a slower proliferation rate compared to lactic acid bacteria, it is difficult to have proliferation of bifidobacteria at the time of producing fermented milk which contains lactic acid bacteria and bifidobacteria. Some types of bifidobacteria do not survive with no or little proliferation if they are co-present with lactic acid bacteria. Accordingly, in the case of producing fermented milk containing lactic acid bacteria and bifidobacteria, it is necessary to increase the addition amount of bifidobacteria or to add galactooligosaccharides described in Patent Literature 2.

### Citation List

### Patent Literature

Patent Literature 1: JP 2722110 B2
Patent Literature 2: JP 2009-189374 A

### Summary of Invention

### Technical Problem

However, for a case in which a high addition amount of bifidobacteria is employed to produce fermented milk containing lactic acid bacteria and bifidobacteria, there is a problem that higher production cost is required as large amounts of bifidobacteria need to be cultured in advance and the large amounts of bifidobacteria need to be added to raw material milk. To obtain the galactooligosaccharides described in Patent Literature 2, there is a problem that higher production cost is required as an additional step is necessary.

An object solved by the present invention is to produce fermented milk containing lactic acid bacteria and bifidobacteria while the number of the live bifidobacteria in the milk are increased and/or maintained by a simple method.

### Solution to Problem

The present invention has the following technical constitutions, and solves the problems accordingly.

(1) A method for producing fermented milk in which a first step for mixing raw material milk, lactic acid bacteria, and bifidobacteria and a second step for fermenting the raw material milk are carried out in order, wherein a step for adding lactase to the raw material milk (lactase addition step) is carried out before the completion of the second step.
(2) The method for producing fermented milk according to (1), wherein the lactase addition step is carried out at one or more time point selected from before the first step, almost simultaneously with the first step, or after the first step.
(3) The method for producing fermented milk according to (1) or (2), wherein lactose contained in the raw material milk is slowly decomposed by the lactase.
(4) The method for producing fermented milk according to any one of (1) to (3), wherein final concentration of the lactase added to the raw material milk is 1.3 unit/g or higher.
(5) The method for producing fermented milk according to (3), wherein glucose and galactose generated by decomposition of lactose is assimilated by the lactic acid bacteria or the bifidobacteria.
(6) The method for producing fermented milk according to (3) or (5), wherein the lactose is decomposed by the lactase and also, in parallel with the decomposition, is assimilated by at least one of the lactic acid bacteria and the bifidobacteria.
(7) The method for producing fermented milk according to (1), wherein the lactase is slowly and/or gradually inactivated during the second step.
(8) Fermented milk including lactic acid bacteria, bifidobacteria, and lactase, wherein the lactase is neutral lactase and present in an inactivated state in the fermented milk and the lactose in the fermented milk is present at 2.0% by mass or less based on the total amount of the fermented milk.
(9) The fermented milk according to (8), wherein the lactose is present at 0.5% by mass or less based on the total amount of the fermented milk.

### Advantageous Effects of Invention

According to the present invention, it is possible to produce fermented milk containing lactic acid bacteria and bifidobacteria while the number of the live bifidobacteria in the milk are increased and/or maintained by a simple method.

### Brief Description of Drawings

Fig. 1 is a drawing illustrating that a tendency of having increasing pH during fermentation is recognized according to the present invention (the pH upon the termination of the fermentation does not change regardless of whether the present invention is carried out or not).
Fig. 2 is a drawing illustrating that an increase ratio of the number of bifidobacteria changes depending on the final concentration of lactase.

### Description of Embodiments

The present invention relates to a method for producing fermented milk in which a first step for mixing raw material milk, lactic acid bacteria, and bifidobacteria and a second step for fermenting the raw material milk are carried out in order, wherein a step for adding lactase to the raw material milk (lactase addition step) is carried out before the completion of the second step.

By carrying out the lactase addition step before the completion of the second step, lactose contained in the raw material milk can be decomposed into glucose and galactose by an enzyme reaction of the lactase. Through the lactase addition step, the amount of lactose contained in the raw material milk is reduced and the amount of glucose contained in the milk and the amount of galactose contained in the milk increase. As a result, the number of live bifidobacteria increases in the fermented milk. The completion of the second step has the same meaning as the completion of fermentation step.

The above effect is not obtained just by adding glucose and/or galactose to raw material milk. In order to increase the number of live bifidobacteria in fermented milk, it is important to increase, after reducing the amount of lactose contained in the raw material milk by adding lactase, the amount of glucose and the amount of galactose.

With regard to the first step, as long as raw material milk, lactic acid bacteria, and bifidobacteria are admixed with one another, the order for admixing is not limited. When raw material milk is mainly used, it is preferable that lactic acid bacteria and bifidobacteria are added to the raw material milk.

The means for mixing raw material milk, lactic acid bacteria, and bifidobacteria is not particularly limited. It is possible that mixing is carried out for just a short time such that lactic acid bacteria and bifidobacteria can almost uniformly distribute in raw material milk. After raw material milk have the almost uniform distribution, the lactic acid bacteria and bifidobacteria may distribute on the bottom surface of the container filled with the raw material milk.

Because the oxygen resistance of bifidobacteria is not high, it is preferable that the time for mixing is as short as possible. A slow mixing rate is preferable in that the amount of oxygen dissolved in raw material milk can be reduced. The amount of the dissolved oxygen can be reduced by increasing the temperature of the raw material milk. It is also important for the milk not to foam during stirring. Furthermore, for reducing or removing the dissolved oxygen, a deaeration operation and/or an aeration operation with inert gas like nitrogen is also effective.

The second step is a step for fermenting the raw material milk by the lactic acid bacteria and the bifidobacteria.

The fermentation temperature for the second step is preferably a temperature at which lactic acid bacteria and bifidobacteria can grow.

Although it may vary depending on the type of lactic acid bacteria and bifidobacteria to be used, the fermentation temperature is preferably within a range of from 20°C to 50°C, and more preferably within a range of from 25°C to 45°C.

If the fermentation temperature is lower than the lower limit, the fermentation can be easily delayed so that it may become difficult to obtain fermented milk with excellent economic efficiency.

On the other hand, if the fermentation temperature is higher than the upper limit, there is a problem that lactic acid bacteria and bifidobacteria, or bifidobacteria may not survive.

The fermentation time for the second step depends on the type of lactic acid bacteria and bifidobacteria to be used and the fermentation temperature for the second step. However, it is preferably within a range of from 1 hour to 48 hours. Because the pH of the raw material milk decreases in accordance with the progress of the fermentation, the pH and/or the pH decrease can be also employed as an indicator.

If the fermentation time is shorter than the lower limit, the fermentation may not sufficiently progress so that it may become difficult to obtain desired fermented milk.

On the other hand, if the fermentation time is longer than the upper limit, there is a possibility that an increase in production cost and/or poor quality of finished fermented milk is caused.

It is preferable that the lactase addition step is carried out at one or more time point selected from before the first step, almost simultaneously with the first step, or after the first step. The lactase addition step may be carried out at two or more time points. After the lactase addition step, the decomposition of lactose contained in raw material milk occurs (lactase reaction).

From the viewpoint of simplifying of the method and obtaining and/or enhancing the effect of proliferating bifidobacteria, it is preferable to carry out the lactase addition step before the first step or almost simultaneously with the first step. Glucose and galactose generated by the decomposition of lactose can be assimilated by the above lactic acid bacteria or the above bifidobacteria.

By adding lactase before the first step or almost simultaneously with the first step, the growth of lactic acid bacteria is suppressed during the fermentation so that the effect of proliferating the bifidobacteria becomes higher. For a case in which lactase is added, the suppressed growth of lactic acid bacteria during the fermentation can be confirmed from that the pH of raw material milk is less likely to decrease.

From the viewpoint of further increasing the effect of proliferating bifidobacteria, it is preferable that the lactase addition step is carried out almost simultaneously with the first step. In accordance with the progress of the fermentation, the decomposition rate of the lactose contained in the raw material milk can be slowed down, and as a result, in the case of having co-presence of the bifidobacteria and the lactic acid bacteria, the bifidobacteria can be provided with an environment for easy assimilation so that the effect of proliferating the bifidobacteria can be increased.

As for the lactase, it is preferable to use neutral lactase. When neutral lactase is used for producing fermented milk, due to a pH decrease in accordance with the progress of the fermentation, the neutral lactase is slowly and/or gradually inactivated. Namely, the decomposition rate of the lactose contained in the raw material milk by the neutral lactase slowly and/or gradually decreases in accordance with the progress of the fermentation. This activity of the neutral lactase can provide, in the case of having co-presence of bifidobacteria and lactic acid bacteria, an environment for easy assimilation of saccharides in the raw material milk by the bifidobacteria so that the number of the bifidobacteria is increased in the fermented milk.

In the present invention, the expression "almost simultaneously" has a relative meaning which varies depending on a growth rate of lactic acid bacteria and bifidobacteria to be used, and it means that at least one of the lactic acid bacteria and the bifidobacteria to be used is in an induction phase. In order to increase the effect of proliferating the bifidobacteria, it is preferable to carry out the lactase addition step when both the lactic acid bacteria and the bifidobacteria are in an induction phase.

The temperature for lactase reaction with raw material milk is preferably 0°C to 55°C, and more preferably 5 to 50°C.

If the temperature for lactase reaction is lower than the lower limit, the decomposition of lactose may be insufficient. To solve such problem, a necessity to extend the lactase reaction time occurs, and thus it becomes difficult to produce fermented milk with efficiency.

On the other hand, if the lactase reaction temperature is higher than the upper limit, lactase is easily inactivated, and thus a state in which the decomposition of lactose is insufficient may be yielded. Furthermore, for having co-presence of lactic acid bacteria and bifidobacteria, it becomes difficult to increase the bifidobacteria.

The time for the lactase reaction with the raw material milk is preferably 0.5 hour to 48 hours, and more preferably 1 hour to 40 hours. When neutral lactase is used as lactase, the neutral lactase is slowly and/or gradually inactivated in accordance with a pH decrease of the raw material milk during the second step, and thus the lactase reaction time is shorter than the second step.

If the lactase reaction time is shorter than the lower limit, the decomposition of lactose may be insufficient.

On the other hand, if the lactase reaction time is longer than the upper limit, there is a problem relating to increased production cost.

The pH of the raw material milk for lactase reaction with the raw material milk is preferably 2.0 to 10.0. The pH is more preferably 2.5 to 9.0, and particularly preferably 3.0 to 8.0. When neutral lactase is used, the pH for the lactase reaction is preferably 5.0 to 8.0. When acidic lactase is used, the pH for the lactase reaction is preferably 3.0 to 7.0.

If the pH is lower than the lower limit or higher than the upper limit, the lactase is easily and/or quickly inactivated so that a state in which the decomposition of lactose is insufficient may occur.

In accordance with the progress of the fermentation, the pH of the raw material milk is lowered. When lactase is neutral lactase, an inactivation of the lactase is caused in accordance with the pH decrease of the raw material milk.

The fermented milk according to the present invention is fermented milk containing lactic acid bacteria, bifidobacteria, and lactase, in which the number of the lactic acid bacteria is within a range of from 1 × 10⁴ to 1 × 10¹⁰ per mL and the number of the bifidobacteria is within a range of from 1 × 10⁴ to 1 × 10¹² per mL.

By following the aforementioned method for producing fermented milk, the number of the lactic acid bacteria and the number of the bifidobacteria contained in the fermented milk can be set within the above range. The number of the lactic acid bacteria and the bifidobacteria contained in the fermented milk means the number of the live bacteria.

It is preferable that at least part of the lactase contained in the fermented milk is neutral lactase. The neutral lactase is preferably present in an inactivated state in the fermented milk.

Even when the neutral lactase is inactivated, the protein structure of the lactase is maintained. As such, by carrying out electrophoresis for the fermented milk itself or a concentrate of the fermented milk, the presence or absence of the neutral lactase in the fermented milk can be confirmed. It is also possible that, after carrying out electrophoresis, the amino acid sequence is elucidated from a specific band obtained from the electrophoresis. Although the sequence of the neutral lactase varies depending on its origin, as it is already known, the presence or absence of the lactase can be determined from the result of the electrophoresis and the amino acid sequence. Similarly, by carrying out electrophoresis for the fermented milk itself or a concentrate of the fermented milk, the presence or absence of acidic lactase in the fermented milk can be confirmed. The amino acid sequence can be also similarly elucidated.

The inactivation of the lactase contained in the fermented milk can be determined by carrying out the means for measuring the lactase activity which will be described later.

The pH of the fermented milk is preferably 5.5 or less, and more preferably 5.0 or less. The lower limit of the pH of the fermented milk is preferably 3.0 or more, and more preferably 3.5 or more.

When the pH of the fermented milk is higher than the upper limit, coagulation of the milk may be insufficient. On the other hand, when the pH of the fermented milk is excessively low, an acidic flavor is too strong, thus easily yielding disrupted flavor balance.

The lactose decomposition rate of the fermented milk is preferably 50% or higher, more preferably 70% or higher, and even more preferably 90% or higher. The upper limit is not limited, but it is 100%, for example. The lactose decomposition rate is expressed as a value which is obtained by dividing the lactose amount before the fermentation with the lactose amount after the fermentation followed by multiplying with 100. The lactose decomposition rate indicates the decreased amount of the lactose, and thus it corresponds to the total of the amount of the lactose decomposed by the lactase and the assimilation amount of the lactose by the lactic acid bacteria and the bifidobacteria. The lactose decomposition rate of the fermented milk which is obtained without undergoing the lactase addition step (the lactic acid bacteria and the bifidobacteria are contained in the fermented milk) is 40% at the maximum, although it may vary depending on raw material milk or bacterial strain to be used, fermentation conditions, or the like. When the fermentation progresses favorably, the lactose decomposition rate is about 30 to 40%.

By carrying out the lactase addition step before the first step or almost simultaneously with the first step, the lactose decomposition rate of the fermented milk by the lactase can be increased.

If the lactose decomposition rate is lower than the lower limit, the insufficient proliferation of the bifidobacteria may occur in the fermented milk containing the lactic acid bacteria and the bifidobacteria.

The lactose amount contained in the fermented milk obtained after the lactase addition step is preferably 2.0% by mass or less, more preferably 1.0% by mass or less, and even more preferably 0.5% by mass or less, based on the total amount of the fermented milk. The lower limit is not limited, but it is 0% by mass, for example. The lactose amount contained in the fermented milk which is obtained without undergoing the lactase addition step is as low as 3.0%, although it may vary depending on raw material milk or bacterial strain to be used, fermentation conditions, or the like. Thus, by measuring the lactose amount contained in the fermented milk, it is possible to determine whether the lactase addition step is carried out or not.

If the lactose amount contained in the fermented milk is higher than the upper limit, the bifidobacteria may not sufficiently proliferate in the fermented milk containing the lactic acid bacteria and the bifidobacteria.

The amount of the glucose contained in the fermented milk which is obtained by undergoing the lactase addition step is preferably 0.5% or more based on the total amount of the fermented milk. It is more preferably 1.0% or more, and even more preferably 2.0% or more. Because of the amount of the glucose contained in the fermented milk at the above amount or more, an environment for easy assimilation of the glucose by the bifidobacteria can be provided even in a state of having co-presence of the lactic acid bacteria and the bifidobacteria.

The amount of the glucose in the fermented milk which has been obtained without undergoing the lactase addition step (the lactic acid bacteria and the bifidobacteria are contained in the fermented milk) is almost zero if fermentation progresses favorably. The glucose is assimilated by the lactic acid bacteria, but due to no assimilation by the bifidobacteria, their proliferation is limited.

The amount of the galactose contained in the fermented milk which has been obtained by undergoing the lactase addition step is preferably 1.0% or more based on the total amount of the fermented milk. It is more preferably 1.5% or more, and even more preferably 2.0% or more. Because of the amount of the galactose contained in the fermented milk at the above amount or more, an environment for easy assimilation of the glucose by the bifidobacteria can be provided even in a state of having co-presence of the lactic acid bacteria and the bifidobacteria.

The amount of the galactose in the fermented milk which has been obtained without undergoing the lactase addition step (the lactic acid bacteria and the bifidobacteria are contained in the fermented milk) is less than 1.0% when fermentation progresses favorably. Because galactose can be assimilated easily by the lactic acid bacteria, the assimilation by the bifidobacteria is difficult, and thus their proliferation is limited.

Hereinbelow, the materials constituting the fermented milk according to the present invention are explained.

### <Raw material milk>

The raw material milk is preferably milk containing lactose, and animal milk like cow milk, ewe milk, or goat milk, human milk, or powder milk obtained by drying them can be used either singly, or a mixture of them can be used, for example. In the present invention, a product obtained by further adding lactose and water to them is also included in the raw material milk.

When the fermented milk is 100% by mass, the raw material milk is preferably 90% by mass or more, more preferably 95% by mass or more, and even more preferably 98% by mass or more.

In the present invention, the milk until the completion of the fermentation is referred to as raw material milk, and the milk after the completion of fermentation is referred to as fermented milk.

With regard to the means for sterilizing raw material milk, it is sufficient to have conditions at which the microorganisms present in the raw material milk and other components described below can be sterilized, and the sterilization means is not limited. Examples of the sterilization means include a means of sterilizing for a short time (for several seconds) at ultra high temperature, a means of sterilizing for a relatively short time (for several minutes) at high temperature, and a means of sterilizing for a long time (for several minutes to several tens of minutes) at low temperature (several tens of Celsius degrees).

### <Lactic acid bacteria>

As for the lactic acid bacteria, microorganisms belonging to the genus Lactococcus, the genus Lactobacillus, or the genus Streptococcus can be exemplified.

Examples thereof include Lactococcus lactis, Lactococcus lactis subsp. cremoris, Lactobacillus casei, Lactobacillus gasseri, Lactobacillus rhamnosus, Lactobacillus acidophilus, Lactobacillus plantarum, Lactobacillus delbrueckii subsp. bulgaricus, and Streptococcus thermophilus. Those lactic acid bacteria may be used either singly or in combination of two or more types thereof.

Furthermore, the fermented milk obtained by use of Lactobacillus delbrueckii subsp. bulgaricus and Streptococcus thermophilus among the above lactic acid bacteria corresponds to yoghurt in narrow sense.

The addition amount of lactic acid bacteria to raw material milk is preferably 1 × 10⁴ to 1 × 10¹⁰ per mL.

If the addition amount is lower than the lower limit, a problem of decreased fermentation rate occurs.

If the addition amount is higher than the upper limit, a problem of increased production cost occurs.

### <Bifidobacteria>

As for the bifidobacteria, microorganisms belonging to the genus Bifidobacterium can be exemplified.

Examples thereof include Bifidobacterium adolescentis, Bifidobacterium bifidum, Bifidobacterium longum ssp. infantis, Bifidobacterium breve, Bifidobacterium longum, and Bifidobacterium animalis ssp. lactis. Those bifidobacteria may be used either singly or in combination of two or more types thereof.

Among them, BB-12 strain (manufactured by Chr. Hansen A/S) of Bifidobacterium animalis ssp. lactis is preferable in that it has acid resistance. Furthermore, as the BB-12 strain also has oxygen resistance at a certain level, it can be easily used for production of fermented milk.

The addition amount of bifidobacteria to raw material milk is preferably 1 × 10⁴ to 1 × 10¹² per mL.

If the addition amount is lower than the lower limit, a problem of deficient number of bacteria occurs.

If the addition amount is higher than the upper limit, a problem of increased production cost occurs.

The ratio of the number of bifidobacteria to the number of lactic acid bacteria that are added to raw material milk is preferably within a range of from 0.01 to 100000. It is more preferably within a range of from 0.1 to 10000, and even more preferably within a range of from 1 to 1000.

If the ratio is lower than the lower limit, it may become difficult to increase the number of the bifidobacteria after the fermentation.

If the ratio is higher than the upper limit, the increased number of the bifidobacteria can be obtained, but the production cost tends to increase, and therefore undesirable.

### <Lactase>

Lactase has an activity of decomposing lactose into galactose and glucose. Lactase is also referred to as β-galactosidase. As for lactase, there are lactase originating from bacteria, lactase originating from yeast, and lactase originating from mold. As for lactase, neutral lactase having the optimum pH in neutral range, or acidic lactase having the optimum pH in acidic range can be used.

Lactase can be used either singly or in combination of two or more types thereof. It is also possible that neutral lactase is used either singly or in combination of two or more types thereof, or acidic lactase is used either singly or in combination of two or more types thereof.

As for the neutral lactase, lactase originating from Kluyveromyces lactis, or lactase originating from Kluyveromyces fragilis, Kluyveromyces marxianus, or Bacillus circulans is preferable. The lactase originating from the genus Kluyveromyces includes lactase deriving from the lactase originating from Kluyveromyces lactis as well as the lactase originating from Kluyveromyces bacteria itself. The optimum pH for having the activity is 6.0 to 7.5, and also the inactivation pH is from 5.5 to 4.0. Because the pH of the fermented milk may be 5.0 or less, when neutral lactase is used, the neutral lactase contained in the fermented milk may be in an inactivated state.

As for the acidic lactase (β-galactosidase), lactase originating from Aspergillus oryzae, lactase originating from Aspergillus kawachii, or lactase originating from Aspergillus niger is preferable.

The addition amount of the lactase added to the raw material milk is, in terms of the final concentration, preferably within a range of from 0.1 to 100 unit/g, and more preferably within a range of from 0.5 to 50 unit/g. It is even more preferably within a range of from 1.3 to 40 unit/g, and particularly preferably within a range of from 1.5 to 30 unit/g. In the present invention, the "unit" may be abbreviated as "U".

If the addition amount is lower than the lower limit, sufficient lactose decomposition is not obtained so that it is difficult to obtain and/or enhance the effect of increasing bifidobacteria.

If the addition amount is higher than the upper limit, the lactose decomposition progresses rapidly so that it is difficult to obtain and/or enhance the effect of increasing bifidobacteria.

### <Others>

It is possible to add saccharides other than lactose such as glucose or galactose, proteins, carbohydrates, lipids, vitamins, minerals, organic acids, organic bases, fruit extracts, flavors, or the like to the raw material milk or fermented milk.

In the case of adding those materials, the addition can be made after carrying out a sterilization treatment in advance or a sterilization treatment can be carried out after adding them to raw material milk.

Hereinbelow, the present invention is explained by using examples, but the present invention is not limited to them.

In the following examples, GODO-YNL2 (neutral lactase) manufactured by GODO SHUSEI Co., Ltd. was used as lactase, unless specifically described otherwise. BB 12 strain manufactured by Chr. Hansen A/S was used as bifidobacteria, unless specifically described otherwise.

### <Means for evaluation>

### (Means for measuring activity of neutral lactase)

The activity of neutral lactase is the activity of lactase (neutral) (β-galactosidase) at pages 801 to 802 of FCC, 4th edition, July 1st 1996, and it was measured by using published activity.

### (Means for measuring activity of acidic lactase)

The activity of acidic lactase is the activity of lactase (acidic) (β-galactosidase) at pages 802 to 803 of FCC, 4th edition, July 1st 1996, and it was measured by using published activity.

### (Means for measuring number of lactic acid bacteria)

For measuring the number of lactic acid bacteria, 0.1 mL of the raw material milk before fermentation or a dilution thereof with physiological saline solution and 0.1 mL of the produced fermented milk or a dilution thereof with physiological saline solution were cultured in "Eiken", which is a BCP-added agar medium for plate count manufactured by Eiken Chemical Co., Ltd. After the culture for 2 days at 37°C, the number of the bacterial cells was measured.

### (Means for measuring number of bifidobacteria)

For measuring the number of bifidobacteria, 0.1 mL of the raw material milk before fermentation or a dilution thereof with physiological saline solution and 0.1 mL of the produced fermented milk per se or a dilution thereof with physiological saline solution were cultured in TOS propionic acid agar medium manufactured by Eiken Chemical Co., Ltd. After the anerobic culture for 2 days at 37°C, the number of the bacterial cells was measured.

### (Means for saccharide analysis)

To 200 µL of milk sample (raw material milk added with various materials or fermented milk described below), 50 µL of 20% sulfosalicylic acid and 800 µL of purified water were added. Then, centrifuge was carried out at 20000 G for 10 minutes. The supernatant was subjected to HPLC analysis (Alliance 2695, manufactured by Waters). For the analysis column, CARBOSep CHO-620 CA (manufactured by Transgenomic Inc.) was used, and the analysis was carried out using a differential refractive index detector with column temperature of 85°C and flow rate of 0.5 mL per minute with purified water as a mobile phase.

### [Example 1]

2 Parts by mass of commercially available skim milk (manufactured by Morinaga Milk Industry Co., Ltd.) and 98 parts by mass of commercially available milk (manufactured by Meiji Dairies Corporation) were mixed and dissolved with each other followed by sterilization treatment at 100°C for 5 minutes. The raw material milk was cooled to 40°C, added with 0.1 part by mass of lactase (the final concentration of 5 U/g), and then subjected to the lactase reaction for 20 minutes at 40°C without any stirring. Then, a lactase inactivation treatment was carried out therefor for 5 minutes at 100°C. The obtained raw material milk was cooled to 43°C, and after being added with each of 0.1 mg of lactic acid bacteria (YF-L812 strain: mixture of Streptococcus thermophilus and Lactobacillus delbrueckii subsp. bulgaricus, manufactured by Chr. Hansen A/S)/1 mL of the raw material milk, and 0.05 mg of bifidobacteria/1 mL of the raw material milk, it was fermented for 4 hours at 43°C to obtain the fermented milk of Example 1 (lactase pre-treatment).

### [Comparative Example 1]

Fermented milk without having any lactase treatment was obtained as follows, simultaneously with Example 1.

2 Parts by mass of commercially available skim milk (manufactured by Morinaga Milk Industry Co., Ltd.) and 98 parts by mass of commercially available milk (manufactured by Meiji Dairies Corporation) were mixed and dissolved with each other followed by sterilization treatment at 100°C for 5 minutes. After the sterilization treatment, the raw material milk was cooled to 43°C, and by adding lactic acid bacteria and bifidobacteria thereto in the same manner as Example 1 followed by fermentation for 4 hours at 43°C, the fermented milk of Comparative Example 1 was obtained.

### <Evaluation> Effect of proliferating YF-L812 strain and BB-12 strain in added milk with lactase pre-treatment

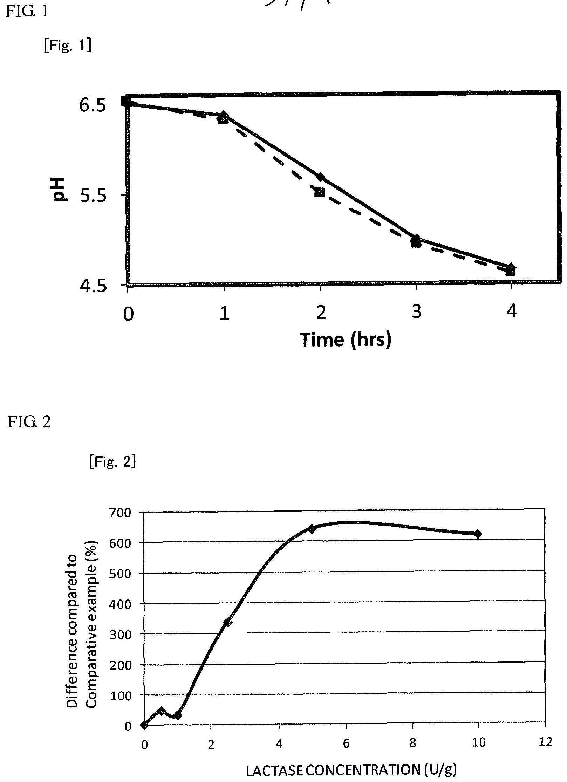

As shown in Table 1, BB-12 strain exhibited the favorable proliferation during the fermentation in Example 1 in which the lactase treatment has been carried out. The difference between Example 1 and Comparative Example 1 at the fermentation end point was about 39% (increase ratio of the number of bifidobacteria in Example - increase ratio of the number of bifidobacteria in Comparative Example which has been carried out simultaneously with Example). In terms of the pH after the fermentation, no difference was seen between Example 1 and Comparative Example 1, and there was no influence on the production of the fermented milk. As shown in Fig. 1, a tendency of having the increasing pH during the fermentation was recognized from Example 1 (the bold line in Fig. 1) compared to Comparative Example 1 (the broken line in Fig. 1). As shown in Table 4, the number of the lactic acid bacteria after the fermentation represents the favorable proliferation from Example 1 in which the lactase treatment has been carried out.

In Table 1, the expression "before fermentation" indicates a value which is measured right after mixing the various materials. The same holds true for other tables.

As shown in Table 2, the lactose decomposition rate after the fermentation was 72% in Example 1. Compared to Comparative Example 1 in which almost no glucose and almost no galactose was contained, there was an increase in the amount of the glucose and the amount of the galactose contained in the fermented milk of Example 1. Because it is considered that glucose or galactose as a monosaccharide can be more easily assimilated than lactose as a disaccharide, it is suggested that the fermented milk of Example 1 was in a state in which bifidobacteria can easily grow.

### [Table 1]

**[Table 1]**

| | Lactase treatment | Before fermentation | | | After fermentation | | | Increase ratio of number of bifidobacteria (%) | **Difference compared to Comparative Example (%)** |
|---|---|---|---|---|---|---|---|---|---|
| | | Number of bifidobacteria (number of cells/mL) | Number of lactic acid bacteria (number of cells/mL) | pH | Number of bifidobacteria (number of cells/mL) | Number of lactic acid bacteria (number of cells/mL) | pH | | |
| Comparative Example 1 | None | 4.0×10⁷ | 1.4×10⁷ | 6.53 | 1.3×10⁸ | 9.6×10⁸ | 4.46 | 325 | - |
| Example 1 | With treatment Pre-treatment | 4.4×10⁷ | 1.5×10⁷ | 6.5 | 1.6×10⁸ | 1.3×10⁹ | 4.48 | 364 | 39 |

### [Table 2]

**[Table 2]**

| | Before fermentation | After fermentation | | |
|---|---|---|---|---|
| | Lactose (%) | Lactose (%) | Glucose (%) | Galactose (%) |
| Comparative Example 1 | 6. 1 | 3.7 | 0. 0 | 0.0 |
| Example 1 | 4.3 | 1.2 | 1.5 | 1.6 |

### [Example 2]

2 Parts by mass of commercially available skim milk (manufactured by Morinaga Milk Industry Co., Ltd.) and 98 parts by mass of commercially available milk (manufactured by Meiji Dairies Corporation) were mixed and dissolved with each other followed by sterilization treatment at 100°C for 5 minutes. After the sterilization treatment, the obtained raw material milk was cooled to 43°C, and after being added with each of 0.05 parts by mass of lactase (the final concentration: 2.5 U/g) and 0.1 mg of lactic acid bacteria (YF-L812 strain)/1 mL of the raw material milk, and 0.05 mg of bifidobacteria/1 mL of the raw material milk, it was fermented for 4 hours at 43°C to obtain the fermented milk of Example 2 (simultaneous addition of lactase).

### [Comparative Example 2]

Simultaneously with Example 2, the fermented milk of Comparative Example 2, in which no lactase treatment was carried out, was obtained in the same manner as Comparative Example 1.

As shown in Table 3, the favorable proliferation of BB-12 strain was exhibited during the fermentation in Example 2 in which the lactase treatment has been carried out. The difference at the fermentation end point between Example 2 and Comparative Example 2 was about 158%. In terms of the pH and the number of the lactic acid bacteria after the fermentation, there was no difference between Example 2 and Comparative Example 2, and there was no influence on the production of the fermented milk.

Although it is not illustrated, a tendency of having the increasing pH during the fermentation was also recognized from Example 2 and Comparative Example 2 like Example 1 and Comparative Example 1.

Because the lactase activity was not recognized from the fermented milk of Example 2, it is suggested that the neutral lactase contained in the fermented milk was inactivated.

As shown in Table 4, the lactose decomposition rate after the fermentation was found to be 91%. It is considered that, by carrying out the lactase addition step almost simultaneously with the first step, the progress of the lactose decomposition by the lactase is obtained in the second step.

### [Table 3]

**[Table 3]**

| | Lactase treatment | Before fermentation | | | After fermentation | | | Increase ratio of number of bifidobacteria (%) | **Difference compared to Comparative Example (%)** |
|---|---|---|---|---|---|---|---|---|---|
| | | Number of bifidobacteria (number of cells/mL) | Number of lactic acid bacteria (number of cells/mL) | pH | Number of bifidobacteria (number of cells/mL) | Number of lactic acid bacteria (number of cells/mL) | pH | | |
| Comparative Example 2 | None | 3.8×10⁷ | 1.0×10⁷ | 6.63 | 9.2×10⁷ | 1.1×10⁹ | 4.65 | 242 | - |
| Example 2 | With treatment Simultaneous addition | 3.5×10⁷ | 1.1×10⁷ | 6.63 | 1.4×10⁸ | 1.1×10⁹ | 4.65 | 400 | **158** |

### [Table 4]

**[Table 4]**

| | Before fermentation | After fermentation | | |
|---|---|---|---|---|
| | Lactose (%) | Lactose (%) | Glucose (%) | Galactose (%) |
| Comparative Example 2 | 6. 0 | 4.2 | 0.0 | 0. 5 |
| Example 2 | 4.3 | 0.4 | 2.0 | 2.2 |

### [Example 3]

According to a conventional manner, Aspergillus oryzae RIB40 was grown on a 4% malt extract (manufactured by Oriental Yeast Co., Ltd.) agar medium. To a 500mL conical flask containing 100 mL of Czapek-Dox broth (manufactured by Difco Laboratories), 5 mm angle of the end part of the grown microbe was inoculated followed by culture for 7 days at 30°C. Then, the culture supernatant was obtained as crude enzyme of acidic lactase. The crude enzyme solution was recovered as a precipitate by adding ammonium sulfate to have 80% saturation, and the obtained precipitate was suspended in 10 mM phosphate buffer solution (pH 6.5). Then, the sample dialyzed against the same buffer solution was used as acidic lactase originating from mold.

The fermented milk of Example 3 was obtained in the same manner as Example 2 except that the above acidic lactase originating from mold was used instead of the lactase used in Example 2.

### [Example 4]

The fermented milk of Example 4 was obtained in the same manner as Example 2 except that neutral lactase originating from Bacillus circulans (Lactoles L3 manufactured by Amano Enzyme Inc.) was used instead of the lactase used in Example 2.

### [Comparative Example 3]

Simultaneously with Examples 3 and 4, the fermented milk not subjected to the lactase treatment was obtained in the same manner as Comparative Example 1.

As shown in Table 5, the favorable proliferation of the BB-12 strain was exhibited during the fermentation in Example 3 in which the treatment with the lactase originating from the mold has been carried out and also in Example 4 in which the treatment with the neutral lactase originating from Bacillus circulans has been carried out. The difference at the fermentation end point between Example 3 and Comparative Example 3 with no lactase treatment was about 97%, and the difference was 144% between the Example 4 and Comparative Example 3.

The acidic lactase activity was recognized from the fermented milk of Example 3. Because the neutral lactase activity was not recognized from the fermented milk of Example 4, it is suggested that the neutral lactase contained in the fermented milk was inactivated.

### [Table 5]

**[Table 5]**

| | Lactase treatment | Number of bifidobacteria before fermentation (number of cells/mL) | Number of bifidobacteria after fermentation (number of cells/mL) | Increase ratio of number of bifidobacteria (%) | **Difference compared to Comparative Example (%)** |
|---|---|---|---|---|---|
| Comparative Example 3 | None | 3.7×10⁷ | 9.7×1 0⁷ | 262 | - |
| Example 3 | With treatment Simultaneous addition Originating from mold | 3.9×10⁷ | 1.4×10⁸ | 359 | **97** |
| Example 4 | With treatment Simultaneous addition Originating from Bacillus | 3.2×10⁷ | 1.3×10⁸ | 406 | **144** |

### [Example 5]

The fermented milk of Example 5 was obtained in the same manner as Example 1 except that lactic acid bacteria (YC-380 strain: mixture of Streptococcus thermophilus and Lactobacillus delbrueckii subsp. bulgaricus, manufactured by Chr. Hansen A/S) was used instead of the lactic acid bacteria (YF-L812 strain) used in Example 1.

### [Comparative Example 4]

Simultaneously with Example 5, the fermented milk not subjected to the lactase treatment was obtained in the same manner as Comparative Example 1 except that lactic acid bacteria (YC-380 strain: mixture of Streptococcus thermophilus and Lactobacillus delbrueckii subsp. bulgaricus, manufactured by Chr. Hansen A/S) was used instead of the lactic acid bacteria (YF-L812 strain) used therein.

As shown in Table 6, the favorable proliferation of BB-12 strain was exhibited during the fermentation in Example 5 in which the treatment with the lactase has been carried out. The difference at the fermentation end point compared to Comparative Example 4 was about 39%.

Because the lactase activity was not recognized from the fermented milk of Example 5, it is suggested that the neutral lactase contained in the fermented milk was inactivated.

Compared to Comparative Example 4 in which almost no amount of glucose or almost no amount of galactose was contained, there was an increase in the amount of the glucose and the amount of the galactose that were contained in the fermented milk of Example 5. Because it is considered that glucose or galactose as a monosaccharide can be more easily assimilated than lactose as a disaccharide, it is suggested that the fermented milk of Example 5 was in a state in which bifidobacteria can easily grow.

### [Table 6]

**[Table 6]**

| | Lactase treatment | Number of bifidobacteria before fermentation (number of cells/mL) | Number of bifidobacteria after fermentation (number of cells/mL) | Increase ratio of number of bifidobacteria (%) | **Difference compared to Comparative Example (%)** |
|---|---|---|---|---|---|
| Comparative Example 4 | None | 5.5×10⁷ | 9.0×10⁷ | 164 | - |
| Example 5 | With treatment Pre-treatment | 6.4×1 0⁷ | 1.3×10 ⁸ | 203 | **39** |

### [Table 7]

**[Table 7]**

| | Before fermentation | After fermentation | | |
|---|---|---|---|---|
| | Lactose (%) | Lactose (%) | Glucose (%) | Galactose (%) |
| Comparative Example 4 | 6. 2 | 3.9 | 0. 2 | 0.8 |
| Example 5 | 2.0 | 0. 9 | 1. 7 | 1.9 |

### [Example 6]

The fermented milk of Example 6 was obtained in the same manner as Example 2 except that lactic acid bacteria (YC-380 strain) was used instead of the lactic acid bacteria (YF-L812 strain) used in Example 2.

### [Comparative Example 5]

Simultaneously with Example 6, the fermented milk not subjected to the lactase treatment was obtained in the same manner as Comparative Example 4.

As shown in Table 8, the favorable proliferation of the BB-12 strain was exhibited during the fermentation by the sample for which the treatment with lactase has been carried out. The difference at the fermentation end point compared to no treatment with lactase was about 465%.

Because the lactase activity was not recognized from the fermented milk of Example 6, it is suggested that the neutral lactase contained in the fermented milk was inactivated.

Compared to Comparative Example 5 in which almost no amount of glucose or almost no amount of galactose was contained, there was an increase in the amount of the glucose and the amount of the galactose that were contained in the fermented milk of Example 6. Because it was considered that glucose or galactose as a monosaccharide can be more easily assimilated than lactose as a disaccharide, it is suggested that the fermented milk of Example 6 was in a state in which bifidobacteria can easily grow.

### [Table 8]

**[Table 8]**

| | Lactase treatment | Number of bifidobacteria before fermentation (number of cells/mL) | Number of bifidobacteria after fermentation (number of cells/mL) | Increase ratio of number of bifidobacteria (%) | **Difference compared to Comparative Example (%)** |
|---|---|---|---|---|---|
| Comparative Example 5 | None | 4.3×10⁷ | 8.7×10⁷ | 202 | **-** |
| Example 6 | With treatment Simultaneous addition | 4.2×10⁷ | 2.8×10⁸ | 667 | **465** |

### [Table 9]

**[Table 9]**

| | Before fermentation | After fermentation | | |
|---|---|---|---|---|
| | Lactose (%) | Lactose (%) | Glucose (%) | Galactose (%) |
| Comparative Example 5 | 6.1 | 3.8 | 0.1 | 0.8 |
| Example 6 | 6.1 | 0.1 | 2.1 | 2.6 |

### [Example 7]

The fermented milk of Example 7 was obtained in the same manner as Example 6 except that the final concentration of the lactase was adjusted from 0.5 U/g to 10 U/g in a stepwise manner.

### [Comparative Example 6]

Simultaneously with Example 7, the fermented milk not subjected to the lactase treatment was obtained in the same manner as Comparative Example 4.

As shown in Table 10, the favorable proliferation of the BB-12 strain was exhibited during the fermentation by the sample for which the treatment with lactase has been carried out. The difference at the fermentation end point compared to no treatment with the lactase was about 32% to 642%.

The obtained results are shown in Fig. 2. As shown in Fig. 2, the difference in terms of the increase ratio of the number of the bifidobacteria compared to Comparative Example was, when the final concentration of the lactase was 0.5 U/g or more and 1.0 U/g or less, just slightly improved compared to Comparative Example in which no lactase has been added. When the final concentration of the lactase was 1.0 U/g or more and 5 U/g or less, a result showing almost linear increase was obtained in the lactase addition concentration dependent manner. When the final concentration of the lactase was more than 5 U/g, the effect depending on the addition concentration could not be confirmed, and it was almost the same effect as the effect with the final lactase concentration of 5 U/g.

Because the neutral lactase activity was not recognized from any fermented milk of Example 7, it is suggested that the neutral lactase contained in the fermented milk was inactivated.

### [Table 10]

**[Table 10]**

| | Lactase treatment | Number of bifidobacteria before fermentation (number of cells/mL) | Number of bifidobacteria after fermentation (number of cells/mL) | Increase ratio of number of bifidobacteria (%) | **Difference compared to Comparative Example (%)** |
|---|---|---|---|---|---|
| Comparative Example 6 | None | 4.3×10⁷ | 5.0×10⁷ | 116 | **-** |
| Example 7 | With treatment 0.5U/g Simultaneous addition | 4. 3×10⁷ | 7.0 ×10⁷ | 163 | **47** |
| | With treatment 1 U/g Simultaneous addition | 4. 6×1 0⁷ | 6.8×10⁷ | 148 | **32** |
| | With treatment 2.5U/g Simultaneous addition | 4.2×10⁷ | 1. 9×10⁸ | 452 | **336** |
| | With treatment 5U/g Simultaneous addition | 3.7×10⁷ | 2.8×10 ⁸ | 758 | **642** |
| | With treatment 10U/g Simultaneous addition | 3.4×10⁷ | 2.5×10⁸ | 735 | **619** |

### [Example 8]

The fermented milk of Example 8 was obtained in the same manner as Example 6 except that the bifidobacteria was changed from BB-12 strain to Bifidobacterium breve JCM 1192.

### [Comparative Example 7]

Simultaneously with Example 8, the fermented milk not subjected to the lactase treatment was obtained in the same manner as Comparative Example 4.

As shown in Table 11, the favorable proliferation of the JCM 1192 strain was exhibited during the fermentation by the sample for which the treatment with the lactase has been carried out. The difference at the fermentation end point compared to no treatment with lactase was about 46%.

Because the neutral lactase activity was not recognized from the fermented milk of Example 8, it is suggested that the neutral lactase contained in the fermented milk was inactivated.

### [Table 11]

**[Table 11]**

| | Lactase treatment | Number of bifidobacteria before fermentation (number of cells/mL) | Number of bifidobacteria after fermentation (number of cells/mL) | Increase ratio of number of bifidobacteria (%) | **Difference compared to Comparative Example (%)** |
|---|---|---|---|---|---|
| Comparative Example 7 | None | 6.1×10⁸ | 3.9 ×10⁸ | 64 | **-** |
| Example 8 | With treatment Simultaneous addition | 5.0×10⁸ | 5.5×10⁸ | 110 | **46** |

### [Example 9]

The fermented milk of Example 8 was obtained in the same manner as Example 6 except that the bifidobacteria was changed from BB-12 strain to Bifidobacterium longum JCM 1217.

### [Comparative Example 8]

Simultaneously with Example 9, the fermented milk not subjected to the lactase treatment was obtained in the same manner as Comparative Example 4.

As shown in Table 12, a decrease in JCM 1192 strain during the fermentation was suppressed in Example 9 in which the lactase treatment has been carried out. The difference at the fermentation end point compared to no treatment with lactase was about 4%.

Because the neutral lactase activity was not recognized from the fermented milk of Example 9, it is suggested that the neutral lactase contained in the fermented milk was inactivated.

### [Table 12]

**[Table 12]**

| | Lactase treatment | Number of bifidobacteria before fermentation (number of cells/mL) | Number of bifidobacteria after fermentation (number of cells/mL) | Increase ratio of number of bifidobacteria (%) | **Difference compared** to **Comparative Example (%)** |
|---|---|---|---|---|---|
| Comparative Example 8 | None | 7.8×10⁸ | 3.5×10⁸ | 45 | **-** |
| Example 9 | With treatment Simultaneous addition | 7.7×10⁸ | 3.8×10⁸ | 49 | **4** |

### [Reference Example 1]

The fermented milk not subjected to the lactase treatment was obtained in the same manner as Comparative Example 4 except that, to raw material milk, glucose and/or galactose was added at 1% by mass, respectively, during the first step.

### [Comparative Example 9]

Simultaneously with Reference Example 1, the fermented milk not subjected to the lactase treatment was obtained in the same manner as Comparative Example 4.

As shown in Table 13, the favorable proliferation of the BB-12 strain was not exhibited during the fermentation in Reference Example 1 in which the fermentation was carried out according to the addition of the saccharide, and almost the same tendency as Comparative Example 9 was shown from Reference Example 1.

### [Table 13]

**[Table 13]**

| | Addition of saccharide | Number of bifidobacteria before fermentation (number of cells/mL) | Number of bifidobacteria after fermentation (number of cells/mL) | Increase ratio of number of bifidobacteria (%) | **Difference compared to Comparative Example (%)** |
|---|---|---|---|---|---|
| Comparative Example 9 | No addition | 5.1×10⁷ | 1.0×10⁸ | 196 | **-** |
| Reference Example 1 | 1% Glucose | 5.2×10⁷ | 8.0×10⁷ | 154 | **-42** |
| | 1% Galactose | 5.0 ×10⁷ | 9.8×10⁷ | 196 | **0** |
| | 1% Glucose + 1% Galactose | 4.5×10⁷ | 6.8×10⁷ | 151 | **-45** |

### [Example 10]

The fermented milk of Example 10 was obtained in the same manner as Example 6 except that the final concentration of the lactase was adjusted to low concentration of 1.3 U/g or 1.5 U/g in a stepwise manner.

### [Comparative Example 10]

Simultaneously with Example 10, the fermented milk not subjected to the lactase treatment was obtained in the same manner as Comparative Example 4.

As shown in Table 14, the favorable proliferation of the BB-12 strain was exhibited during the fermentation by the sample for which the treatment with the lactase has been carried out. The difference at the fermentation end point compared to no treatment with the lactase was about 97% to 107%. When this numerical value of difference and the numerical value of the final lactase concentration are applied to Fig. 2, they can be plotted at a position that is close to the curve. Considering that the number of the bifidobacteria before the fermentation was higher in Example 10 compared to Example 7 so that the nutritional source was easily depleted and the pH decrease was easily caused, it is believed that the fermentation was completed even before having a sufficient increase of the number of the bifidobacteria. As such, it is suggested that, when the final concentration of the lactase was 1.3 U/g or 1.5 U/g, the number of the bifidobacteria increases in the lactase addition concentration dependent manner.

Because the lactase activity was not recognized from any fermented milk of Example 10, it is suggested that the neutral lactase contained in the fermented milk was inactivated.

### [Table 14]

**[Table 14]**

| | Lactase treatment | Number of bifidobacteria before fermentation (number of cells/mL) | Number of bifidobacteria after fermentation (number of cells/mL) | Increase ratio of number of bifidobacteria (%) | **Difference compared to Comparative Example (%)** |
|---|---|---|---|---|---|
| Comparative Example 10 | None | 1.2×10⁸ | 2.8×10⁸ | 233 | **-** |
| Example 10 | With treatment 1.3U/g Simultaneous addition | 1. 0×10⁸ | 3.3×10⁸ | 330 | **97** |
| | With treatment 1.5U/g Simultaneous addition | 1.0×10⁸ | 3.4×10⁸ | 340 | **107** |

Based on the above, it is demonstrated that, to obtain and/or enhance the effect of proliferating bifidobacteria in fermented milk containing lactic acid bacteria and the bifidobacteria, a simple adjustment of the amount of glucose or galactose in the milk is insufficient, and decreasing the amount of lactose and also increasing the amount of glucose and galactose by addition of lactase are important. To further enhance the effect of proliferating bifidobacteria in fermented milk containing lactic acid bacteria and bifidobacteria, in particular, it is shown be favorable that the lactase addition step is carried out almost simultaneously with the first step.

With regard to fermented milk containing lactic acid bacteria and bifidobacteria, it is shown that the number of live bifidobacteria can be maintained by having the lactase addition step even if there is a tendency that the bifidobacteria do not survive.

## Claims

1. A method for producing fermented milk in which a first step for mixing raw material milk, lactic acid bacteria, and bifidobacteria and a second step for fermenting the raw material milk are carried out in order, wherein a step for adding lactase to the raw material milk (lactase addition step) is carried out before the completion of the second step.

2. The method for producing fermented milk according to claim 1, wherein the lactase addition step is carried out at one or more time point selected from before the first step, almost simultaneously with the first step, or after the first step.

3. The method for producing fermented milk according to claim 1 or 2, wherein lactose contained in the raw material milk is slowly decomposed by the lactase.

4. The method for producing fermented milk according to any one of claims 1 to 3, wherein final concentration of the lactase added to the raw material milk is 1.3 unit/g or higher.

5. The method for producing fermented milk according to claim 3, wherein glucose and galactose that are generated by decomposition of lactose is assimilated by the lactic acid bacteria or the bifidobacteria.

6. The method for producing fermented milk according to claim 3 or 5, wherein the lactose is decomposed by the lactase and also, in parallel with the decomposition, is assimilated by at least one of the lactic acid bacteria and the bifidobacteria.

7. The method for producing fermented milk according to claim 1, wherein the lactase is slowly inactivated during the second step.

8. Fermented milk comprising lactic acid bacteria, bifidobacteria, and lactase, wherein the lactase is neutral lactase and present in an inactivated state in the fermented milk and lactose is present at 2.0% by mass or less.

9. The fermented milk according to claim 8, wherein the lactose is present at 0.5% by mass or less.
